# EUROPÄISCHE PATENTSCHRIFT

(11) **EP 1 065 998 B1**
(45) Veröffentlichungstag und Bekanntmachung des Hinweises auf die Patenterteilung: **26.04.2006**
(21) Anmeldenummer: 99914563.4
(22) Anmeldetag: 25.03.1999
(51) Int. Cl.: A61F 2/36, A61F 2/34

(54) **GELENKPROTHESE MIT KLEMMSITZ-VERBINDUNG ZWISCHEN PROTHESENKOMPONENTEN**
JOINT PROSTHESIS WITH PRESS FIT CONNECTION BETWEEN PROSTHETIC COMPONENTS
PROTHESE ARTICULAIRE AVEC ASSEMBLAGE PAR COMPRESSION DES COMPOSANTS

(30) Priorität: 25.03.1998 DE 19813074
(43) Veröffentlichungstag der Anmeldung: 10.01.2001
(73) Patentinhaber: CeramTec AG Innovative Ceramic Engineering, 73207 Plochingen (DE)
(72) Erfinder: VON CHAMIER, Wilfried, D-70193 Stuttgart (DE); HOCH, Ernst, D-73274 Notzingen (DE)
(74) Vertreter: Uppena, Franz
(86) Internationale Anmeldenummer: PCT/EP1999/002245
(87) Internationale Veröffentlichungsnummer: WO 1999/048446

(56) Entgegenhaltungen:
- EP-A- 0 597 553
- EP-A- 0 648 478
- EP-A- 0 864 303
- DE-A- 2 548 077
- DE-A- 19 611 248
- US-A- 4 697 325
- US-A- 5 362 311

## Beschreibung

Die Erfindung betrifft eine Klemmsitz-Verbindung zwischen Prothesenkomponenten von Gelenk-Prothesen entsprechend dem Oberbegriff des ersten Anspruchs.

Gelenk-Prothesen, bei denen ein Gelenkpartner als Pfanne und der andere Gelenkpartner als Kugelkopf ausgebildet ist, der in der Pfanne drehbar gelagert ist, sind insbesondere als Schulter- und Hüftgelenk-Prothesen bekannt. Diese Prothesen sind in der Regel modular aufgebaut. Hüftgelenk-Endoprothesen beispielsweise bestehen aus der Pfanne, die in den Hüftknochen, und dem Schaft, der in den Oberschenkelknochen eingesetzt wird. Die Pfanne besteht in der Regel aus einer metallischen Außenschale, in die eine Einsatzschale aus Keramikwerkstoff oder einem biokompatiblen Kunststoff eingesetzt wird. Als Verbindungstechnik wird hier die Klemmsitz-Verbindung angewendet. Eine Klemmsitz-Verbindung ist beispielsweise aus der DE 196 11 248 A1 bekannt. Der Schaft hat einen Zapfen, den sogenannten Konus, auf den der Kugelkopf aufgesteckt wird. Bei den modular aufgebauten Endoprothesen werden zur Anpassung an den Körperbau des Patienten lmplantatkomponenten unterschiedlicher Materialien und Größe miteinander verbunden. Beispielsweise werden Kugelköpfe aus einer Kobalt-Chrom-Legierung oder aus einer Aluminiumoxid-Keramik auf einen Konus aus Titan aufgesteckt. Als Verbindungstechnik zwischen metallischen oder keramischen Kugelköpfen und dem Konus wird hier ebenfalls die Klemmsitz-Verbindung angewendet, insbesondere die konische Klemmung. Dabei wird der Kugelkopf mit einer konischen Bohrung auf den Konus gesetzt. Nach dem Aufstecken des Kugelkopfs auf den Konus erfolgt die Fixierung durch einen Schlag auf den Kugelkopf.

Wie aus der Veröffentlichung "Das Prinzip der Konus-Steckverbindung für keramische Kugelköpfe bei Hüftendoprothesen" von G. Willmann, Mat.-wiss. u. Werkstofftech. 24, 315-319 (1993), bekannt ist, sind alle Keramiken spröde, das heißt empfindlich gegen nichtflächig eingeleitete Belastungen, also Spannungskonzentrationen. Die Kontaktflächen zwischen der Bohrung in der Keramikkugel und der Oberfläche des Konus müssen optimal aufeinander abgestimmt sein. Es muß ein maximal möglicher Traganteil der Flächen vorhanden sein. Erreicht werden könnte dies durch extrem hohe Anforderungen an die Maßtoleranzen der Bohrung und des metallischen Konus. Da das aber technisch nicht realisierbar und wirtschaftlich nicht sinnvolt ist, wird das Potential der plastischen Verformbarkeit der Metalle ausgenutzt, indem man die Oberfläche des metallischen Konus strukturiert. Die Oberfläche wird zum Beispiel stark aufgerauht oder gezielt mit einer Struktur, beispielsweise einer Rille, versehen. Wird jetzt der Kugelkopf auf den metallischen Konus gesteckt, verformt sich die strukturierte Oberfläche und der Traganteil der Flächen wird vergrößert. Dadurch wird die Spannungskonzentration minimiert und zusätzlich wird die Reibung erhöht, so daß auch die Verdrehsicherheit gefördert wird. Je größer die im Mikrometerbereich liegende Rauhtiefe ist, desto höher ist die ertragbare Bruchlast. Da der Keramikwerkstoff aber härter ist als der metallische Werkstoff des Konus, kann, insbesondere bei belastungsbedingten Relativbewegungen zwischen Konus und Kugelkopf, Abrieb auf der Oberfläche des Konus auftreten und eine Lockerung der Verbindung eintreten. Verschleißerscheinungen durch Relativbewegungen sind auch bei Gelenkpfannen möglich, bei denen die Einsatzschale mittels Klemmsitz in der Außenschale gehalten wird.

In der DE-OS 25 48077 wird eine Gelenkendoprothese vorgestellt, wobei die Erfindung die Befestigung einer Gelenkkugel aus gesinterter Oxidkeramik auf einem konischen Zapfen eines in dem Femur einsetzbaren Schaftteils betrifft. Auf den konischen Zapfen, den Vaterkonus, wird mittels Preßsitz die Gelenkkugel aufgesetzt, die eine entsprechende Aussparung, den Mutterkonus, aufweist. Im Oberflächenbereich des oxidkeramischen Mutterkonus kann eine Metallisierungsschicht aus körperverträglichen Metallen aufgebracht sein. Über eine mögliche Aufraung der Metallisierungsschicht wird nichts ausgesagt. Der verformbare Oberflächenbereich des Vaterkonus besteht aus dem Werkstoff des Konus selbst, nicht aus einer Beschichtung, und wird durch gewindeartige Rillen, Wellen oder napfartige Vertiefungen gebildet. Auf den Vaterkonus selbst wird keine verformbare Schicht aufgetragen.

In der US-PS 5,362,311 wird eine Verbindung zwischen der Gelenkkugel und dem konischen Zapfen des in den Femurteil einsetzbaren Schaftes beschrieben, wobei in die konische Öffnung der Gelenkkugel eine fingerhutähnliche Metallhülse gesteckt wird, die in sich selbst oder auf ihrer Oberfläche strukturiert sein kann. Zur Unterstützung der Klemmwirkung ist die Wanddicke der Hülse über ihre Länge unterschiedlich und zwar ist die Wand am Ende des Zapfens dicker als an seinem Fuß.

Aus der DE 196 11 248 A1 ist eine prothetische Gelenkpfanne mit einer Außenschale bekannt, in der mit Klemmsitz eine austauschbare Einsatzschale eingesetzt ist. Über die Beschaffenheit der Klemmflächen wird hier keine Aussage gemacht.

Aufgabe der vorliegenden Erfindung ist es, eine verbesserte Klemmsitz-Verbindung zwischen den Prothesenkomponenten von Gelenk-Prothesen vorzustellen.

Die Lösung der Aufgabe erfolgt mit Hilfe der kennzeichnenden Merkmale des ersten Anspruchs. Vorteilhafte Ausgestaltungen der Erfindung werden in den Unteransprüchen beansprucht.

Die erfindungsgemäße Beschichtung aus einem biokompatiblen Metall oder einer biokompatiblen Metallegierung auf einer der Klemmflächen der Komponenten, der Innenfläche der konischen Bohrung des Kugelkopfs beziehungsweise dem Außenkonus-Abschnitt der Einsatzschale, wirkt als Haftvermittler zwischen Kugelkopf und Konus des Schaftes beziehungsweise Einsatzschale und Außenschale der Gelenkpfanne. Die Beschichtung läßt sich in jeder gewünschten Dicke herstellen. Denkbar ist eine elektrolytische Abscheidung oder eine Abscheidung aus der Gasphase (Sputtern). Ein bevorzugtes Auftragsverfahren ist das Aufspritzen, wobei sich insbesondere für das Spritzen hochschmelzender Metalle oder Metallegierungen das Plasmaspritzen eignet. Das Plasmaspritzen bietet die Möglichkeit, aufgrund der Einstellungen von Flammenabstand und Flammentemperatur eine Beschichtung in der erforderlichen Dicke herzustellen. Wichtig bei allen Beschichtungsverfahren und Beschichtungswerkstoffen ist, daß zwischen dem Beschichtungswerkstoff und der Oberfläche der Prothesenkomponente aus Keramik oder Kunststoff eine gute Haftung besteht. Diese kann durch eine mechanischen Verklammerung in der rauhen Oberfläche des Keramikwerkstoffs oder Kunststoffs oder durch eine Verbindung des Auftragswerkstoffs mit dem entsprechenden Werkstoff in der Grenzschicht erfolgen. Letzteres ist insbesondere beim Plasmaspritzen hochschmelzender metallischer Werkstoffe möglich, beispielsweise bei Titanlegierungen.

Beim Aufpressen des Kugelkopfs auf den Konus sowie der Einsatzschale in die Außenschale kann neben der Reibhaftung durch den großen Druck auf die Beschichtung eine Verbindung zwischen dem Werkstoff der Beschichtung und dem metallischen Werkstoff der Klemmfläche des Konus beziehungsweise der Außenschale auftreten, beispielsweise in Form einer sogenannten Kaltverschweißung, mittels derer die Haftung der beiden Komponenten erhöht werden kann.

Die Haftung der Prothesenkomponenten aus Keramik oder Kunststoff auf ihren Partnern aus Metall, die Haftung eines Kugelkopfs auf einem Konus sowie einer Einsatzschale in einer Außenschale, wird vorteilhaft dadurch erhöht, daß die Beschichtung eine rauhe Oberfläche aufweist. Beim Aufpressen eines Kugelkopfs auf den Konus eines Schaftes sowie einer Einsatzschale in eine Außenschale werden die Spitzen der rauhen Oberfläche der Beschichtung eingeebnet, wodurch ein hoher tragender Flächenanteil für die Reibhaftung erzeugt wird. Die Oberfläche der Beschichtung kann bereits durch das Auftragsverfahren selbst, beispielsweise Plasmaspritzen, rauh sein, kann aber auch durch eine Nachbearbeitung, beispielsweise Sandstrahlen, auf die gewünschte Rauhtiefe gebracht werden.

Durch die Beschichtung entsteht im Bereich der Klemmflächen, auf der Mantelfläche der konischen Bohrung beziehungsweise des Außenkonus-Abschnitts, ein entsprechender Materialzuwachs. Deshalb ist es vorteilhaft, wenn die Abmessungen der Prothesenkomponenten im Bereich der Klemmflächen im Hinblick auf die für einen Klemmsitz erforderlichen Abmessungen mit Beschichtung so gewählt werden, daß die Beschichtung in einer auf die jeweilige Komponentenpaarung abgestimmten optimalen Rauhtiefe aufgetragen werden kann.. Durch den Überzug auf eine der Klemmflächen besteht außerdem vorteilhaft die Möglichkeit, Toleranzen, bzw. Abweichungen von der Geradheit und Rundheit von Konus und konischer Bohrung in dem Kugelkopf beziehungsweise Einsatzschale und Innenkonus-Abschnitt in der Außenschale auszugleichen, wodurch die Gefahr von Relativbewegungen unter Belastung aufgrund schlechter Passung ausgeschlossen wird.

Je nach Werkstoff, Auftragsverfahren und Schichtdicke kann die Beschichtung eine Rauhtiefe von 20 µm bis 90 µm aufweisen. Die Rauhtiefen der Klemmflächen der Komponenten, die durch Haftreibung miteinander verbunden werden sollen, haben einen entscheidenden Einfluß insbesondere auf die Bruchlast der keramischen Komponente. Mit zunehmender Rauhtiefe der Klemmfläche des Metallischen Partners bei herkömmlichen Verbindungen nimmt die Bruchlast der keramischen Komponente zu. Um einen entsprechend der Erfindung festen und haltbaren Sitz der Komponenten zu gewährleisten, sollte vorteilhafterweise die Rauhtiefe der Beschichtung möglichst nicht wesentlich unter 20 µm absinken. Mit abnehmender Rauhtiefe steigt der Widerstand gegen das Einebnen der Spitzen und es muß eine höhere Kraft aufgewendet werden.

Als besonders vorteilhaft hat sich eine Rauhtiefe zwischen 60 µm und 90 µm erwiesen. Erklären läßt sich das so, daß dann Spitzen in einer genügenden Höhe vorhanden sind, die beim mechanischen Aufpressen eines Kugelkopfs auf einen Konus beziehungsweise beim Eindrücken einer Einsatzschale in eine Außenschale eingeebnet werden und mit zunehmender Einebnung einen größerwerdenden, tragenden Flächenanteil ergeben. Mit steigendem Flächenanteil steigt die Reibung und damit auch die Sicherheit gegen Relativbewegungen, insbesondere das Verdrehen, von Kugelkopf und Konus beziehungsweise Einsatzschale und Außenschale.

Kugelköpfe und Einsatzschalen aus Keramikwerkstoff haben sich nicht nur wegen ihrer Verschleißfestigkeit als vorteilhaft erwiesen. Da Keramikwerkstoffe keine korrosive Wirkung auf Metatte oder Metallegierungen ausüben, können sie unbedenklich mit einer Beschichtung aus einem biokompatiblen Metall oder einer biokompatiblen Metallegierung versehen werden.

Weiterhin ist es vorteilhaft, wenn der Werkstoff der Beschichtung und der Werkstoff der Klemmfläche des Konus beziehungsweise der Außenschale übereinstimmen. Es ist bekannt, daß in dem Fall, wenn Implantatkomponenten aus unterschiedlichen Materialien, beispielsweise ein Kugelkopf aus Kobalt-Chrom auf einen Schaft aus Titanlegierungen gesteckt wird, aufgrund von Relativbewegung zwischen den Teilen und der Anwesenheit der wie ein Elektrolyt wirkenden Körperflüssigkeit eine sogenannte Fretting-Korrosion möglich ist. Aufgrund der durch Körperbelastung auftretenden Mikrobewegungen zwischen Kugelkopf und Konus kann ein nachweisbarer Abrieb auftreten, der zu einer Verletzung der schützenden Oxidschicht der ansonsten biokompatiblen Metalle oder Metallegierungen führt. Durch die darauf auftretenden galvanischen Reaktionen der gegeneinanderreibenden unterschiedlichen Metallkomponenten entsteht die sogenannte Fretting-Korrosion.

An Hand einer Hüftgelenk-Endoprothese als Ausführungsbeispiel wird die Erfindung näher erläutert.

Es zeigen:
- Figur 1: den Schaft einer Hüftgelenk-Endoprothese und einen Kugelkopf mit einer mit dem erfindungsgemäßen Überzug versehenen konischen Bohrung, geschnitten,
- Figur 2: den für das Implantieren vorbereiteten Schaft mit dem auf den Konus aufgesteckten Kugelkopf und
- Figur 3: eine Gelenkpfanne, in deren Außenschale eine mit der erfindungsgemäßen Beschichtung versehene Einsatzschale eingepreßt ist, geschnitten.

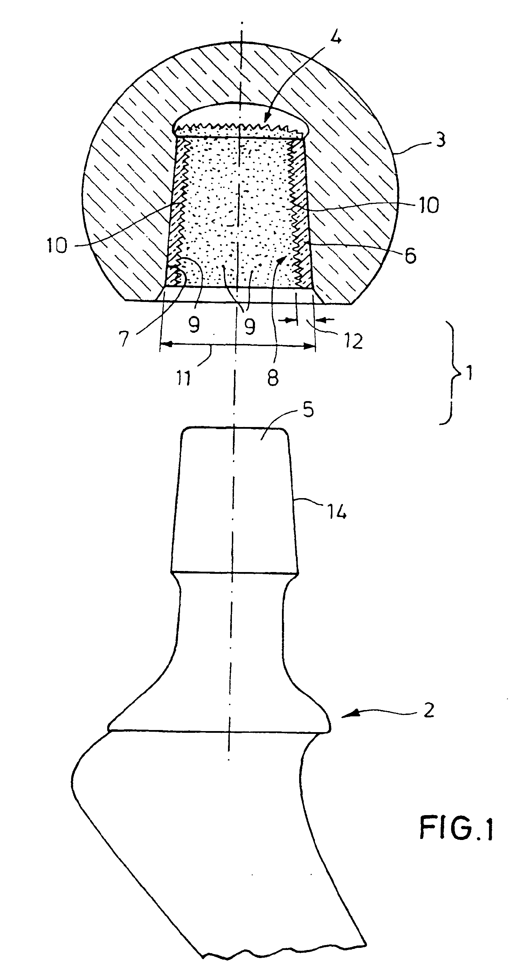

In Figur 1 sind von einer Hüftgelenk-Endoprothese 1 der Schaft 2 und ein zugehöriger Kugelkopf 3 aus Keramik, beispielsweise aus Aluminiumoxid, in vergrößertem Maßstab dargestellt. Der Kugelkopf 3 weist eine konische Bohrung 4 zur Aufnahme des Konus 5 des Schafts 2 auf. Der Schaft 2 und sein Konus 5 sind im vorliegenden Ausführungsbeispiel aus einer Titanlegierung, beispielsweise TiAl6V4, gefertigt. Zur Verdeutlichung ist die erfindungsgemäße Beschichtung 6 auf der Mantelfläche 7 der konischen Bohrung 4, der Klemmfläche des Kugelkops 3, wie sie in der geschnittenen Hälfte des Kugelkopfes 3 zu sehen ist; vergrößert dargestellt. Die Beschichtung 6 besteht aus der gleichen Titanlegierung wie der Schaft und ist durch Flammspritzen aufgetragen worden. Zur Lösung der der Erfindung zugrundeliegenden Aufgabe genügt es, wenn nur eine der Klemmflächen, hier die Mantelfläche 7 der konischen Bohrung 4, mit der Beschichtung 6 versehen ist.

Deutlich ist die rauhe Oberfläche 8 der Beschichtung 6 zu sehen, die durch das Auftragsverfahren, das Flammspritzen, erzeugt wurde. Die Rauhtiefe Rₜ liegt bevorzugt zwischen 60 µm und 90 µm. Die Spitzen 9 werden beim Aufpressen des Kugelkopfs 3 auf den Konus 5 durch die Klemmfläche 14 eingedrückt und füllen die Täler 10 aus. Dadurch erhöht sich der tragende Flächenanteil in der Beschichtung 6 der Klemmfläche 7 des Kugelkopfs 3 beträchtlich.

Damit der Kugelkopf 3 nach dem Aufpressen auf den Konus 5 die richtige Position einnimmt, sind der Durchmesser 11 der konischen Bohrung 4 und die Schichtdicke 12 der Beschichtung 6 so aufeinander abgestimmt, daß auf die Klemmfläche 7 eine Beschichtung 6 in einer vorgebbaren Rauhtiefe und Dicke 12 aufgetragen werden kann, so daß die für den Zusammenhalt der Komponenten erforderliche Klemmkraft erreicht wird.

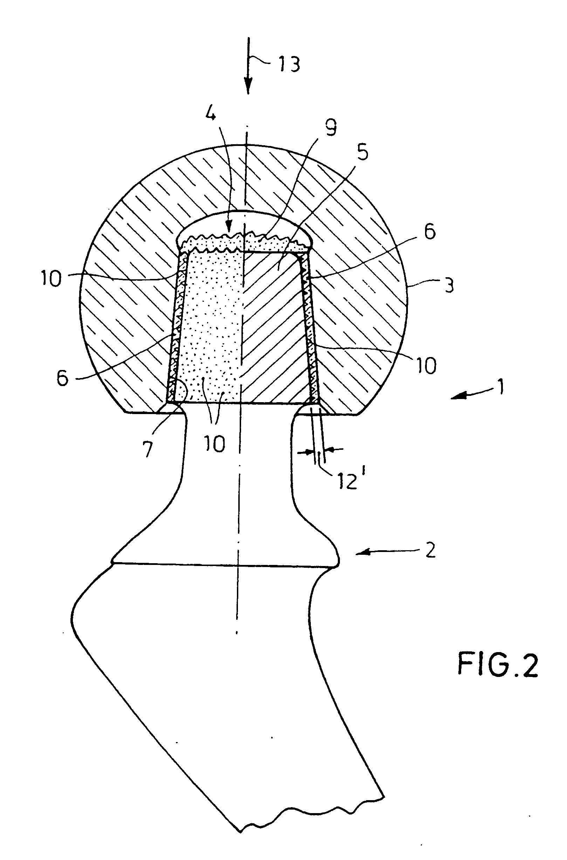

Figur 2 zeigt den Kugelkopf 3 und den Schaft 2 in zusammengesetztem Zustand. Der Kugelkopf 3 ist in Pfeilrichtung 13, beispielsweise durch einen Hammerschlag, auf den Konus 5 aufgepreßt worden. Dabei wurden die Spitzen 9 (Figur 1) beim Gleiten über die Klemmfläche 14 eingeebnet und die Beschichtung 6 auf die Dicke 12' zusammengepreßt, wie in Fig. 2 in der Ansicht und im Schnitt der Beschichtung 6 zu sehen ist. Der Traganteil, im Gegensatz zur Figur 1 in der Figur 2 in der Ansicht des eingeebneten Überzugs weiß dargestellt, wird erhöht und vergleichmäßigt, wodurch die Spannungskonzentrationen, die ansonsten durch die nichtflächig eingeleiteten Belastungen auftreten, auf ein Minimum abgebaut werden. Zusätzlich mit der dadurch bedingten größeren Reibung wird die Verdrehsicherheit der beiden Komponenten erhöht. Die schwarzen Flächen 10 in der Ansicht der Beschishtung 6 stellen hier die verbliebenen, noch nicht aufgefüllten Täler dar. Die aufgrund der hohen Flächenpressung auf die Beschichtung 6 an der Grenzfläche zwischen der Klemmfläche 14 des Konus 5 und der Beschichtung 6 auftretenden metallurgischen Verbindungen, die zur Steigerung der Haftung beitragen, sind nicht dargestellt.

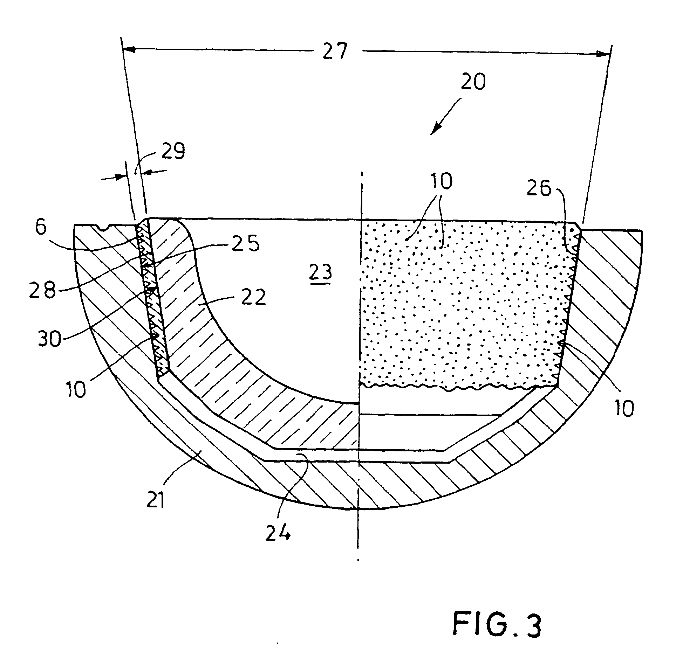

In Figur 3 ist ein pothetische Gelenkpfanne 20 im Schnitt dargestellt. Sie wird von einer Außenschale 21 und einer darin eingesetzten Einsatzschale 22 gebildet. Die Beschichtung und ihre Merkmale sind mit denselben Bezugsziffern versehen wie beim vorhergehenden Ausführungsbeispiel. Die Außenschale 21 besteht aus einem biokompatiblen Metall, während die Einsatzschale 22 aus Keramik oder aus einem biokompatiblen Kunststoff bestehen kann. In der Einsatzschale 22 dient der Hohlraum 23 zur Aufnahme des Kugelkopfs des Gelenks (hier nicht dargestellt, siehe Figuren 1 und 2).

Während die Außenkontur der Außenschale 21 im vorliegenden Ausführungsbeispiel die Form einer Kugel hat, weist der zur Aufnahme der Einsatzschale 22 vorgesehene Hohlraum 24 einen konisch geformten Teil 25 auf. Seine Mantelfläche bildet die Klemmfläche 26 für die Einsatzschale 22, die nur in diesem Bereich der Innenwand an der Außenschale 21 anliegt.

Damit die Einsatzschale 22 nach dem Eindrücken in die konisch geformte Ausnehmung 25 der Außenschale 21 die richtige Position einnimmt, ist der Durchmesser 27 des Außenkonus-Abschnitts 30 der Einsatzschale 22 so zu bemessen, daß auf der Klemmfläche 28 eine Beschichtung 6 in der Dicke und vorgebbaren Rauhtiefe aufgetragen werden kann, mit dem eine für den Zusammenhalt der Komponenten erforderliche Klemmkraft erreicht wird.

Der zusammengebaute Zustand von Außenschale 21 und Einsatzschale 22 zeigt, daß die rauhe Oberfläche der Beschichtung 6 durch die Klemmfläche 26 der Außenschale 21 im wesentlichen eingeebnet worden ist und die Beschichtung 6 auf die Dicke 29 zusammengepreßt worden ist. In der Ansicht der Beschichtung 6 ist, wie in der Figur 2 des vorhergehenden Ausführungsbeispiels, der tragende Anteil der Beschichtung 6 weiß dargestellt. Die schwarzen Flächen 10 sollen auch hier die verbliebenen Täler darstellen. Wie die Ansicht der eingeebneten Beschichtung 6 zeigt, wird ein hoher und gleichmäßig tragender Flächenanteil für die Klemmsitz-Verbindung von Außenschale 21 und Einsatzschale 22 erreicht.

## Patentansprüche

1. Gelenk-Prothese (1, 20), bei der die Gelenkpartner jeweils aus zwei Komponenten zusammengesetzt sind, von denen die eine Komponente (2; 21) metallisch und im Knochen verankerbar ist und die andere, den Reibpartner des Gelenks bildende Komponente (3; 22) aus Keramik oder einem biokompatiblen Kunststoff besteht, wobei die beiden Komponenten durch eine Klemmsitz-Verbindung miteinander verbunden sind, die Komponente (3; 22) aus Keramik oder Kunststoff auf ihrer Klemmfläche (7; 28), über die sie mit der metallischen Komponente (2; 21) verbunden ist, mit einem auf die Klemmfläche (7; 28) aufgetragenen Beschichtung (6) aus einem biokompatiblen Metall oder einer biokompatiblen Metallegierung versehen ist und die Beschichtung (6) eine rauhe Oberfläche (8) zur Herstellung eines Klemmsitzes hat, **dadurch gekennzeichnet, dass** die für einen Klemmsitz erforderliche Rauhtiefe der Beschichtung (6) zwischen 60 µm und 90 µm liegt.

2. Gelenk-Prothese nach Anspruch 1, **dadurch gekennzeichnet, dass** der Werkstoff der Beschichtung (6, 7; 28) dem Werkstoff der metallischen Prothesenkomponente (2; 21) entspricht.

3. Gelenk-Prothese nach einem der Ansprüche 1 oder 2, **dadurch gekennzeichnet, dass** die Beschichtung (6) eine Spritzschicht ist.

## Claims

1. Joint prosthesis (1, 20), in which the joint partners are composed of two respective components, of which the one component (2; 21) is metallic and can be anchored in the bone and the other component (3; 22) forming the friction partner of the joint consists of ceramic material or a biocompatible plastics material, with the two components being connected together by means of a press-fit connection, the component (3; 22) made from ceramic material or plastics material being provided, on its pressing surface (7; 28) by way of which it is connected to the metallic component (2; 21), with a coating (6) that is made from a biocompatible metal or a biocompatible metal alloy that is applied to the pressing surface (7; 28), and the coating (6) having a rough surface (8) in order to establish a press fit, **characterised in that** the roughness-height of the coating (6) that is required for a press fit lies between 60 µm and 90 µm.

2. Joint prosthesis according to claim 1, **characterised in that** the material of the coating (6, 7; 28) corresponds to the material of the metallic prosthesis component (2; 21).

3. Joint prosthesis according to one of claims 1 or 2, **characterised in that** the coating (6) is a spray layer.

## Revendications

1. Prothèse articulaire (1, 20), dans laquelle les éléments associés de l'articulation sont formés chaque fois de deux composants, parmi lesquels l'un des composants (2, 21) est métallique et peut être ancré dans l'os et l'autre composant (3, 22), qui constitue le partenaire de frottement de l'articulation, est en céramique ou en une matière plastique biologiquement compatible, les deux composants étant liés l'un à l'autre par ajustement serré, les composants (3, 22) en céramique ou en matière plastique, au niveau de leur surface de serrage (7, 28) par l'intermédiaire de laquelle ils sont liés aux composants métalliques (2, 21), étant pourvus d'un revêtement (6) en un métal biocompatible ou en un alliage biocompatible appliqué sur la surface de serrage (7, 28) et le revêtement (6) présentant une surface (8) rugueuse aux fins de réaliser l'ajustement serré, **caractérisé par le fait que** la profondeur de rugosité du revêtement (6) nécessaire pour l'ajustement serré est comprise entre 60 µm et 90 µm.

2. Prothèse articulaire selon la revendication 1, **caractérisée par le fait que** le matériau du revêtement (6, 7, 28) correspond au matériau des composants métalliques (2, 21) de la prothèse.

3. Prothèse articulaire selon une des revendications 1 ou 2, **caractérisée par le fait que** le revêtement (6) est une couche appliquée par projection.
